# EUROPÄISCHE PATENTSCHRIFT

(11) **EP 0 600 370 B1**
(45) Veröffentlichungstag und Bekanntmachung des Hinweises auf die Patenterteilung: **17.07.1996**
(21) Anmeldenummer: 93118991.4
(22) Anmeldetag: 25.11.1993
(51) Int. Cl.: C07C 315/02, C07C 317/28, C07C 323/25, C07C 323/41

(54) **Di-(3-(2-chlorethylsulfonyl)-l-propyl)-amin-Hydrochlorid und Verfahren zu seiner Herstellung**
Di-(3-(2-chloroethylsulphonyl)-1-propylamine hydrochloride and method for its preparation
Chlorhydrate de di-(3(2-chloroéthylsulfonyl)-1-propylamine et procédé pour sa préparation

(30) Priorität: 02.12.1992 DE 4240421
(43) Veröffentlichungstag der Anmeldung: 08.06.1994
(73) Patentinhaber: HOECHST AKTIENGESELLSCHAFT, 65929 Frankfurt am Main (DE)
(72) Erfinder: Angenendt, Heinrich, Dr., D-65510 Idstein (DE); Meier, Michael, Dr., D-60487 Frankfurt am Main (DE); Schams, Wolfram, D-65934 Frankfurt am Main (DE)

(56) Entgegenhaltungen:
- EP-A- 0 070 808
- EP-A- 0 518 321
- WO-A-91/13867
- BULLETIN OF THE ACADEMY OF SCIENCES OF THE USSR, DIVISION OF CHEMICAL SCIENCE, Bd. 25, Nr. 4 , Oktober 1976 New York, US, Seiten 919 - 921 A.R. DERZHINSKII, ET AL.: 'Synthesis of 2-chloroethyl alkyl sulphones by the oxidative chlorination of 2-hydroxyethyl alkyl sulphides'

## Beschreibung

Die Erfindung betrifft das Di-(3-(2-chlorethylsulfonyl)-1-propyl)-amin-Hydrochlorid und ein Dreistufenverfahren zu seiner Herstellung über die neuen Verbindungen N,N-Di-(3-(2-hydroxyethylthio)-1-propyl)-carbonsäureamid und Di-(3-(2-hydroxyethylthio)1-propyl)-amin, ausgehend von Diallylcarbonsäureamiden.

Das Di-(3-(2-chlorethylsulfonyl)-1-propyl)-amin-Hydrochlorid ist ein wertvolles Zwischenprodukt zur Herstellung von Reaktivfarbstoffen; die N,N-Di-(3-(2-hydroxyethylthio)1-propyl)-carbonsäureamide und das Di-(3-(2-hydroxyethylthio)-1-propyl)-amin sind die Vorprodukte zur Herstellung des genannten Hydrochlorids.

Gegenstand der Erfindung ist somit ein Verfahren zur Herstellung von Di-(3-(2-chlorethylsulfonyl)-1-propyl)-amin-Hydrochlorid der Formel

HCl·HN(CH₂-CH₂-CH₂-SO₂-CH₂-CH₂-Cl)₂,

indem man a) 1 mol eines N,N-Diallylcarbonsäureamids der allgemeinen Formel (1)

R-CON(CH₂-CH=CH₂)₂ (1)

in welcher R Wasserstoff, Alkyl(C₁-C₆) oder Cycloalkyl(C₄-C₈) bedeutet, mit 4 bis 8 mol, vorzugsweise mit 5 bis 7 mol Mercaptoethanol bei Temperaturen von 0° bis -70°C, vorzugsweise von -40° bis -60°C, in Gegenwart von reinem Sauerstoff oder einem Gemisch von Sauerstoff mit einem inerten Gas oder einem Inertgasgemisch, vorzugsweise in Gegenwart von Luft, gegebenenfalls in einem gegenüber den Reaktionsteilnehmern und den Reaktionsbedingungen inerten organischen Lösungsmittel, bei Atmosphärendruck oder Überdruck umsetzt zum entsprechenden Carbonsäureamid der allgemeinen Formel (2)

R-CON(CH₂-CH₂-CH₂-S-CH₂-CH₂-OH)₂ (2)

in welcher R die vorstehend genannte Bedeutung hat, b) das so erhaltene Carbonsäureamid mit der wäßrigen Lösung eines alkalisch wirkenden Mittels bei Temperaturen von 40° bis 120°C bei einem pH-Wert von 10 bis 14 hydrolysiert zum Di-(3-(2-hydroxyethylthio)-1-propyl)-amin und c) 1 Mol dieses Amins mit 4 bis 6 mol, vorzugsweise 4,1 bis 4,5 mol, gasförmigem Chlor in 2 bis 15 %iger, vorzugsweise 3 bis 10 %iger wäßriger Salzsäure bei Temperaturen von 20° bis 110°C, vorzugsweise 40°C bis 80°C, umsetzt zum Di-(3-(2-chlorethylsulfonyl)1-propyl)-amin-Hydrochlorid.

Zu den drei Verfahrensstufen sei im einzelnen folgendes ausgeführt:
Bei Stufe 1, Umsetzung des N,N-Diallylcarbonsäureamids mit Mercaptoethanol, kann man so vorgehen, daß man die beiden Reaktionspartner mischt, auf Reaktionstemperatur bringt und dann das Reaktionsgemisch mit dem Sauerstoff oder mit dem den Sauerstoff enthaltenden Gemisch in Kontakt bringt, oder so, daß man einen der beiden Reaktionspartner bei Reaktionstemperatur vorlegt und den zweiten in Gegenwart von Sauerstoff oder dem den Sauerstoff enthaltenden Gemisch zudosiert.

Zur Vermeidung von Nebenreaktionen ist es zweckmäßig, das Mercaptoethanol bei der Reaktionstemperatur vorzulegen und das Diallylamid in Gegenwart des Sauerstoffs oder des den Sauerstoff enthaltenden Gemisches zuzudosieren.

Als Inertgase, die einzeln oder im Gemisch zusammen mit Sauerstoff verwendet werden können, seien beispielsweise Stickstoff und Helium genannt.

Die Reaktion kann bei Atmosphärendruck oder Überdruck durchgeführt werden. Die Umsetzung kann ferner in einem inerten organischen Lösungsmittel, wie beispielsweise Toluol oder Ethanol vorgenommen werden; Vorteile können dadurch aber nicht erzielt werden.

Ein Arbeiten bei oberhalb 0°C ist zwar möglich, jedoch kommt es hierbei verstärkt zu Nebenreaktionen.

Nach Reaktionsende destilliert man überschüssiges Mercaptoethanol und gebildete Nebenprodukte ab.

Die in Stufe 2 vorzunehmende Verseifung der in Stufe 1 gebildeten Verbindung der genannten allgemeinen Formel (2) erfolgt zweckmäßigerweise mit der wäßrigen Lösung eines Alkalimetall- oder Erdalkalimetallhydroxyids oder -carbonats, vorzugsweise mit wäßriger Natriumhydroxid- oder Kaliumhydroxidlösung bei den weiter oben genannten Temperaturen und pH-Werten.

In Stufe 3, Umsetzung des in Stufe 2 erhaltenen Di-(3-(2-hydroxyethylthio)-1-propyl)amins mit Chlorgas, geht man zweckmäßigerweise so vor, daß man 1 mol Di-(3-(2-hydroxyethyl)thio-1-propyl)-amin in Wasser löst, mit 20 bis 37 %iger wäßriger Salzsäure neutralisiert, in die Lösung bei den weiter oben genannten Temperaturen die genannten Molmengen Chlor einleitet und nach dem Abkühlen der Lösung das Di-(3-(2-chlorethylsulfonyl)-1-propyl)-amin-Hydrochlorid absaugt.

Setzt man Di-(3-(2-chlorethylsulfonyl)-1-propyl)-amin-Hydrochlorid in bekannter Weise mit dem Kondensationsprodukt von Cyanurfluorid mit 3,6,8-Trisulfonaphthalin-2-azo-(4'-aminobenzol) um, so erhält man den Farbstoff der Formel 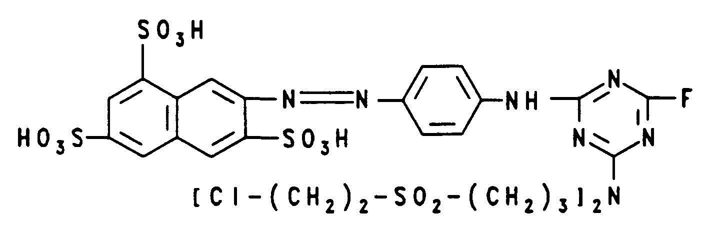
Die nachstehenden Beispiele dienen zur Erläuterung des erfindungsgemäßen Verfahrens, ohne es darauf zu beschränken.

### Beispiel 1 (Reaktionsstufe 1)

125,2 g (1,0 mol) N,N-Diallylformamid werden in 2,5 Stunden bei -50°C unter kräftigem Rühren unter Durchleiten von Luft in 468,8 g (6,0 mol) Mercaptoethanol eindosiert. Anschließend rührt man bis zum Ende der Reaktion weiter und arbeitet dann destillativ auf. Man erhält so 251,1 g N,N-Di-(3-(2-hydroxyethylthio)-1-propyl)-formamid, was einer Ausbeute von 90 % d.Th. entspricht.
¹H-NMR ([D₆]DMSO): δ=1,70, 1,74 (2m; 4H, CH₂-CH₂-CH₂), 2,47, 2,48 (2t, J=7Hz; 4H, S-CH₂-CH₂-OH), 2,56 (t,J=7Hz; 4H, S-CH₂), 3,26, 3,30 (2m; 4H, N-CH₂), 3,52 (t,J=7Hz;4H,CH₂-OH), 4,69 (s; 2H, OH), 8,00 (s; 1H, HCO).

### Beispiel 2 (Reaktionsstufe 1)

139,2 g (1,0 mol) N,N-Diallylacetamid werden in 2 Stunden bei 0°C unter kräftigem Rühren unter Durchleiten von Luft in 468,8 g (6,0 mol) Mercaptoethanol eindosiert. Anschließend rührt man bis zum Ende der Reaktion weiter und arbeitet dann destillativ auf. Man erhält so 209,8 g N,N-Di-(3-(2-hydroxyethylthio)-1-propyl)-acetamid, was einer Ausbeute von 71 % d.Th. entspricht.
¹H-NMR (CDCl₃): δ=1,85, 1,88 (2m; 4H, CH₂-CH₂-CH₂), 2,11 (2; 3H, COCH₃), 2,30 (s; breit; 2H, OH), 2,55, 2,57 (2t, J=7Hz; 4H, S-CH₂-CH₂-OH), 2,73, 2,75 (2t, J=6Hz, 4H, S-CH₂), 3,38, 3,43 (2m; 4H, N-CH₂), 3,73, 3,76 (2t, J=6Hz; 4H, CH₂-OH).

### Beispiel 3 (Reaktionsstufe 1)

139,2 g (1,0 mol) N,N-Diallylacetamid werden in 15 Minuten bei -50°C unter kräftigem Rühren unter Durchleiten von Luft in 468,8 g (6,0 mol) Mercaptoethanol eindosiert. Anschließend rührt man bis zum Ende der Reaktion weiter und arbeitet dann destillativ auf. Man erhält so 271,8 g N,N-Di-(3-(2-hydroxyethylthio)-1-propyl)-acetamid, was einer Ausbeute von 92 % d.Th. entspricht.
Die spektroskopischen Daten sind mit den in Beispiel 2 angegebenen identisch.

### Beispiel 4 (Reaktionsstufe 2)

140,7 g N,N-Di-(3-(2-hydroxyethylthio)-1-propyl)-formamid (0,5 mol) werden in 160 ml 20 %iger Natronlauge bis zur vollständigen Entacylierung zum Rückfluß erhitzt. Man erhält so 121,5 g Di-(3-(2-hydroxyethylthio)-1-propyl)-amin, was einer Ausbeute von 96 % d.Th. entspricht.
¹H-NMR ([D₆]DMSO): δ=1,66,(m; 4H, CH₂-CH₂-CH₂), 2,57, (m; 12H, NCH₂, CH₂SO₂CH₂), 3,58 (t, J=7Hz; 4H, CH₂OH), 4,02 (s, breit; 3H, OH, NH)

### Beispiel 5 (Reaktionsstufe 2)

147,8 g N,N-Di-(3-(2-hydroxyethylthio)-1-propyl)-acetamid (0,5 mol) werden in 160 ml 20 %iger Natronlauge bis zur vollständigen Entacylierung zum Rückfluß erhitzt. Man erhält so 119,4 g Di-(3-(2-hydroxyethylthio)-1-propyl)-amin, was einer Ausbeute von 94 % d.Th. entspricht.
Die spektroskopischen Daten sind mit den in Beispiel 4 angegebenen identisch.

### Beispiel 6 (Reaktionsstufe 3)

126,7 g Di-(3-(2-hydroxyethylthio)-1-propyl)-amin (0,5 mol) werden in 500 ml Wasser gelöst und mit 50 ml 37 %iger Salzsäure versetzt. Anschließend leitet man bei 80°C unter kräftigem Rühren bis zum Ende der Reaktion insgesamt 155 g Chlor ein. Nach Abkühlen auf 0°C saugt man ab und erhält so nach Trocknen im Vakuum 148,8 g Di-(3-(2-chlorethylsulfonyl)-1-propyl)-amin-Hydrochlorid als farblose Kristalle mit Schmelzpunkt 183-185°C, was einer Ausbeute von 76 % d.Th. entspricht.
¹H-NMR([D6]DMSO)δ=2,11 (m; 4H, CH₂CH₂CH₂), 3,03 (t, J=6Hz; 4H, NCH₂), 3,38 (t, J=6Hz; 4H, NCH₂CH₂SO₂), 3,95 (t, J=6Hz; 4H, CH₂-Cl), 9,26 (s; 2H, NH₂⁺Cl⁻).

## Patentansprüche

1. Verfahren zur Herstellung von Di-(3-(2-chlorethylsulfonyl)-1-propyl)-amin-Hydrochlorid, dadurch gekennzeichnet, daß man
a) 1 mol eines N,N-Diallylcarbonsäureamids der allgemeinen Formel (1)
R-CON(CH₂-CH=CH₂)₂ (1)
in welcher R Wasserstoff, Alkyl(C₁-C₆) oder Cycloalkyl(C₄-C₈) bedeutet, mit 4 bis 8 mol Mercaptoethanol bei Temperaturen von 0° bis -70°C in Gegenwart von reinem Sauerstoff oder einem Gemisch von Sauerstoff mit einem inerten Gas oder einem Inertgasgemisch, gegebenenfalls in einem gegenüber den Reaktionsteilnehmern und den Reaktionsbedingungen inerten organischen Lösungsmittel, bei Atmosphärendruck oder Überdruck umsetzt zum Carbonsäureamid der allgemeinen Formel (2)
R-CON(CH₂-CH₂-CH₂-S-CH₂-CH₂-OH)₂ (2)
in welcher R die vorstehend genannte Bedeutung hat,
b) diese Verbindung mit der wäßrigen Lösung eines alkalisch wirkenden Mittels bei Temperaturen von 40° bis 120°C bei einem pH-Wert von 10 bis 14 hydrolysiert und
c) 1 mol des so erhaltenen Di-(3-(2-hydroxyethylthio)-1-propyl)-amins mit 4 bis 6 mol gasförmigem Chlor in 2 bis 15 %iger Salzsäure bei Temperaturen von 20 bis 110°C umsetzt zum Di-(3-(2-chlorethylsulfonyl)-1-propyl)-amin-Hydrochlorid.

2. Verfahren nach Anspruch 1, dadurch gekennzeichnet, daß man 1 mol des N,N-Diallylcarbonsäureamids der dort genannten allgemeinen Formel (1) mit 5 bis 7 mol Mercaptoethanol umsetzt.

3. Verfahren nach mindestens einem der Ansprüche 1 und 2, dadurch gekennzeichnet, daß man das N,N-Diallylcarbonsäureamid mit dem Mercaptoethanol bei Temperaturen von -40° bis -60°C umsetzt.

4. Verfahren nach mindestens einem der Ansprüche 1 bis 3, dadurch gekennzeichnet, daß man das Mercaptoethanol vorlegt und dazu das Diallylamid in Gegenwart des Sauerstoffs oder des den Sauerstoff enthaltenden Gemisches zudosiert.

5. Verfahren nach mindestens einem der Ansprüche 1 bis 4, dadurch gekennzeichnet, daß man das N,N-Diallylcarbonsäureamid mit dem Mercaptoethanol in Gegenwart von Luft umsetzt.

6. Verfahren nach mindestens einem der Ansprüche 1 bis 5, dadurch gekennzeichnet, daß man die Hydrolyse des Carbonsäureamids der in Anspruch 1 genannten allgemeinen Formel (2) mit der wäßrigen Lösung eines Alkalimetall- oder Erdalkalimetallhydroxids oder -carbonats vornimmt.

7. Verfahren nach mindestens einem der Ansprüche 1 bis 6, dadurch gekennzeichnet, daß man die Hydrolyse des Carbonsäureamids der in Anspruch 1 genannten allgemeinen Formel (2) mit wäßriger Natriumhydroxid- oder Kaliumhydroxidlösung vornimmt.

8. Verfahren nach mindestens einem der Ansprüche 1 bis 7, dadurch gekennzeichnet, daß man die Umsetzung des N,N-Diallylcarbonsäureamids mit dem Mercaptolethanol in Toluol oder Ethanol als inertem organischen Lösungsmittel durchführt.

9. Verfahren nach mindestens einem der Ansprüche 1 bis 8, dadurch gekennzeichnet, daß man 1 mol des Di-(3-(2-hydroxyethylthio)-1-propyl)-amins mit 4,1 bis 4,5 mol gasförmigem Chlor umsetzt.

10. Verfahren nach mindestens einem der Ansprüche 1 bis 9, dadurch gekennzeichnet, daß man die Umsetzung des Di-(3-(2-hydroxyethylthio)-1-propyl)-amins mit dem gasförmigen Chlor bei Temperaturen von 40°C bis 80°C vornimmt.

11. Verfahren nach mindestens einem der Ansprüche 1 bis 10, dadurch gekennzeichnet, daß man die Umsetzung des Di-(3-(2-hydroxyethylthio)-1-propyl)-amins mit dem gasförmigen Chlor in 3 bis 10 %iger wäßriger Salzsäure vornimmt.

12. Carbonsäureamide der allgemeinen Formel
R-CON(CH₂-CH₂-CH₂-S-CH₂-CH₂-OH)₂,
in welcher R Wasserstoff, Alkyl(C₁-C₆) oder Cycloalkyl(C₄-C₈) bedeutet.

13. Carbonsäureamid der Formel
H-CON(CH₂-CH₂-CH₂-S-CH₂-CH₂-OH)₂

14. Carbonsäureamid der Formel
CH₃-CON(CH₂-CH₂-CH₂-S-CH₂-CH₂-OH)₂.

15. Verbindung der Formel
HN(CH₂CH₂CH₂-S-CH₂CH₂-OH)₂.

## Claims

1. A process for preparing di(3-(2-chloroethylsulfonyl)-1-propyl)amine hydrochloride, which comprises
a) reacting 1 mol of an N,N-diallylcarboxamide of the formula (1)
R-CON(CH₂-CH=CH₂)₂ (1)
in which R is hydrogen, alkyl(C₁-C₆) or cycloalkyl(C₄-C₈) with 4 to 8 mol of mercaptoethanol at temperatures of 0° to -70°C in the presence of pure oxygen or a mixture of oxygen with an inert gas or an inert gas mixture, if appropriate in an organic solvent which is inert to the reactants and the reaction conditions, at atmospheric pressure or superatmospheric pressure to give the carboxamide of the formula (2)
R-CON(CH₂-CH₂-CH₂-S-CH₂-CH₂-OH)₂ (2)
in which R has the abovementioned meaning,
b) hydrolyzing this compound with the aqueous solution of an alkaline agent at temperatures of 40° to 120°C at a pH of 10 to 14, and
c) reacting 1 mol of the di(3-(2-hydroxyethylthio)-1-propyl)amine thus obtained with 4 to 6 mol of chlorine gas in 2 to 15% hydrochloric acid at temperatures of 20 to 110°C to give di(3-(2-chloroethylsulfonyl)-1-propyl)amine hydrochloride.

2. The process as claimed in claim 1, wherein 1 mol of the N,N-diallylcarboxamide of the formula (1) mentioned there is reacted with 5 to 7 mol of mercaptoethanol.

3. The process as claimed in at least one of claims 1 and 2, wherein the N,N-diallylcarboxamide is reacted with mercaptoethanol at temperatures of -40° to -60°C.

4. The process as claimed in at least one of claims 1 to 3, wherein the mercaptoethanol is introduced first and the diallylamide is metered thereto in the presence of oxygen or the oxygen-containing mixture.

5. The process as claimed in at least one of claims 1 to 4, wherein the N,N-diallylcarboxamide is reacted with mercaptoethanol in the presence of air.

6. The process as claimed in at least one of claims 1 to 5, wherein the hydrolysis of the carboxamide of the formula (2) mentioned in claim 1 is effected using the aqueous solution of an alkali metal hydroxide or alkaline earth metal hydroxide or alkali metal carbonate or alkaline earth metal carbonate.

7. The process as claimed in at least one of claims 1 to 6, wherein the hydrolysis of the carboxamide of the formula (2) mentioned in claim 1 is effected using aqueous sodium hydroxide solution or potassium hydroxide solution.

8. The process as claimed in at least one of claims 1 to 7, wherein the reaction of the N,N-diallylcarboxamide with the mercaptoethanol is carried out in toluene or ethanol as the inert organic solvent.

9. The process as claimed in at least one of claims 1 to 8, wherein 1 mol of di(3-(2-hydroxyethylthio)-1-propyl)amine is reacted with 4.1 to 4.5 mol of chlorine gas.

10. The process as claimed in at least one of claims 1 to 9, wherein the reaction of di(3-(2-hydroxyethylthio)-1-propyl)amine with chlorine gas is carried out at temperatures of 40°C to 80°C.

11. The process as claimed in at least one of claims 1 to 10, wherein the reaction of di(3-(2-hydroxyethylthio)-1-propyl)amine with chlorine gas is carried out in 3 to 10% aqueous hydrochloric acid.

12. A carboxamide of the formula
R-CON(CH₂-CH₂-CH₂-S-CH₂-CH₂-OH)₂,
in which R is hydrogen, alkyl(C₁-C₆) or cycloalkyl(C₄-C₈).

13. The carboxamide of the formula
H-CON(CH₂-CH₂-CH₂-S-CH₂-CH₂-OH)₂

14. The carboxamide of the formula
CH₃-CON(CH₂-CH₂-CH₂-S-CH₂-CH₂-OH)₂.

15. The compound of the formula
HN(CH₂CH₂CH₂-S-CH₂CH₂-OH)₂.

## Revendications

1. Procédé pour la préparation de chlorhydrate de di-(3-(2-chloréthylsulfonyl)-1-propyl)-amine, caractérisé en ce que
a) on fait réagir 1 mole d'un amide d'acide N,N-diallylcarboxylique de formule générale (1)
R-CON(CH₂-CH=CH₂)₂ (1)
dans laquelle R représente l'hydrogène, alkyle en C₁-C₆ ou cycloalkyle en C₄-C₈, avec 4 à 8 moles de mercaptoéthanol à des températures de 0 à -70 °C, en présence de l'oxygène pur ou d'un mélange d'oxygène avec un gaz inerte ou un mélange de gaz inertes, éventuellement dans un solvant organique inerte vis-à-vis des réactants et dans les conditions de réaction, à la pression atmosphérique ou sous une surpression, pour obtenir le carboxamide correspondant de formule générale (2)
R-CON(CH₂-CH₂-CH₂-S-CH₂-CH₂-OH)₂ (2)
dans laquelle R a la signification donnée ci-dessus,
b) on hydrolyse le carboxamide obtenu avec la solution aqueuse d'un agent alcalin, à des températures de 40 à 120 °C, à une valeur de pH de 10 à 14, et
c) on fait réagir 1 mole de la di-(3-(2-hydroxyéthylthio)-1-propyl)-amine ainsi obtenue avec 4 à 6 moles de chlore gazeux dans l'acide chlorhydrique aqueux à 2 à 15 %, à des températures de 20 à 110 °C, pour obtenir le chlorhydrate de di-(3-(2-chloréthylsulfonyl)-1-propyl)-amine.

2. Procédé selon la revendication 1, caractérisé en ce que l'on fait réagir 1 mole du N,N-diallylcarboxamide de formule générale (1) précitée avec 5 à 7 moles de mercaptoéthanol.

3. Procédé selon au moins l'une des revendications 1 et 2, caractérisé en ce que l'on fait réagir le N,N-diallylcarboxamide avec le mercaptoéthanol à des températures de -40 à -60 °C.

4. Procédé selon au moins l'une des revendications 1 et 3, caractérisé en ce que l'on introduit d'abord le mercaptoéthanol et on ajoute ensuite progressivement le diallylamide en présence de l'oxygène ou du mélange contenant l'oxygène.

5. Procédé selon au moins l'une des revendications 1 et 4, caractérisé en ce que l'on fait réagir le N,N-diallylcarboxamide avec le mercaprtoéthanol en présence de l'air.

6. Procédé selon au moins l'une des revendications 1 et 5, caractérisé en ce que l'on met en oeuvre l'hydrolyse du carboxamide de la revendication 1 de formule générale (2) précitée avec la solution aqueuse d'un carbonate ou d'un hydroxyde de métal alcalin ou alcalino-terreux.

7. Procédé selon au moins l'une des revendications 1 et 6, caractérisé en ce que l'on met en oeuvre l'hydrolyse du carboxamide de la revendication 1 de formule générale (2) précitée avec la solution d'hydroxyde de potassium ou d'hydroxyde de sodium.

8. Procédé selon au moins l'une des revendications 1 et 7, caractérisé en ce que l'on met en oeuvre la réaction du N,N-diallylcarboxamide avec le mercaptoéthanol dans du toluène ou de l'éthanol en tant que solvant organique inerte.

9. Procédé selon au moins l'une des revendications 1 et 8, caractérisé en ce que l'on fait réagir 1 mole de la di-(3-(2-hydroxyéthylthio)-1-propyl)-amine avec 4,1 à 4,5 moles de chlore gazeux.

10. Procédé selon au moins l'une des revendications 1 et 9, caractérisé en ce que l'on effectue la réaction de la di-(3-(2-hydroxyéthylthio)-1-propyl)-amine avec le chlore gazeux à des températures de 40 à 80 °C.

11. Procédé selon au moins l'une des revendications 1 et 10, caractérisé en ce que l'on met en oeuvre la réaction de la di-(3-(2-hydroxyéthylthio)-1-propyl)-amine avec le chlore gazeux dans l'acide chlorhydrique aqueux à 3 à 10 %.

12. Carboxamides de formule générale
R-CON(CH₂-CH₂-CH₂-S-CH₂-CH₂-OH)₂,
dans laquelle R représente l'hydrogène, alkyle en C₁-C₆ ou cycloalkyle en C₄-C₈.

13. Carboxamides de formule
H-CON(CH₂-CH₂-CH₂-S-CH₂-CH₂-OH)₂

14. Carboxamides de formule
CH₃-CON(CH₂-CH₂-CH₂-S-CH₂-CH₂-OH)₂.

15. Composé de formule
HN(CH₂CH₂CH₂-S-CH₂CH₂-OH)₂.
